# EUROPEAN PATENT SPECIFICATION

(11) **EP 0 351 586 B1**
(45) Date of publication and mention of the grant of the patent: **18.01.1995**
(21) Application number: 89111482.9
(22) Date of filing: 23.06.1989
(51) Int. Cl.: C12N 15/85, C12N 15/06, C12P 21/02

(54) **Plasmids containing a chicken beta-actin promoter and a human factor VIII:C gene, transformants and processes for preparing human factor VIII:C**
Hühner-beta-Actin-Promoter und menschliches Faktor VIII-C-Gen enthaltende Plasmide, Transformanten und Verfahren zur Herstellung des menschlichen Faktors VIII-C
Plasmides contenant un promoteur du gène codant pour la bêta-actine aviaire et un gène codant pour le facteur VIII-C humain, transformant et méthode pour la préparation de facteur VIII-C humain

(30) Priority: 24.06.1988 JP 157570/88
(43) Date of publication of application: 24.01.1990
(73) Proprietor: Juridical Foundation The Chemo-Sero-Therapeutic Research Institute, Kumamoto-shi Kumamoto-ken (JP); TEIJIN LIMITED, Osaka-shi Osaka-fu (JP)
(72) Inventor: Yonemura, Hiroshi, Kumamoto-shi Kumamoto-ken (JP); Sugiyama, Takashi c/o Biomedical Institute, Hino-shi Tokyo-to (JP); Araki, Masatake, Kumamoto-shi Kumamoto-ken (JP); Nozaki, Chikateru, Kumamoto-shi Kumamoto-ken (JP); Miyazaki, Junichi Higashimachi, Kumamoto-shi Kumamoto-ken (JP); Yamamura, Ken-ichi Higashimachi, Kumamoto-shi Kumamoto-ken (JP)
(74) Representative: VOSSIUS & PARTNER

(56) References cited:
- EP-A- 0 174 608
- WO-A-88/00831
- GENE, vol. 48, no. 1, 13th February 1987, pages 1-11, Elsevier Science Publishers B.V. (Biomedical Division), Amsterdam, NL; N. FREGIEN et al.: "Activating elements in the promoter region of the chicken beta-actin gene"
- NUCLEIC ACIDS RESEARCH, vol. 11, no. 23, 1983, pages 8286-8300, IRL Press Ltd, Oxford, GB; T.A. KOST et al.: "The nucleotide sequence of the chick cytoplasmic beta-actin gene"
- THE JOURNAL OF BIOLOGICAL CHEMISTRY, vol. 264, no. 16, 5th June 1989, pages 9539-9546, US; W.W. QUITSCHKE et al.: "The beta actin promoter. High levels of transcription depend upon a ccaat binding factor"

## Description

The present invention relates to a process for preparing human blood coagulation factor VIII:C (hereinafter referred to as "factor VIII:C") by using a novel expression vector, to said expression vector and to a transformant used in said process. More particularly, the present invention relates to an expression vector wherein a gene of human factor VIII:C is incorporated downstream of a chicken β-actin promoter, to a transformant prepared by transforming an animal cell with said expression vector, and to a process for preparing human factor VIII:C by using said expression vector and said transformant.

Human factor VIII:C is a blood plasma glycoprotein which is involved in the intrinsic pathway of blood coagulation and acts as an accessory factor which accelerates the activation of factor X by activated factor IX, as a cofactor of the activated factor IX. In vivo, factor VIII:C forms a complex with von Willebrand factor and circulates in blood.

In patients of hemophilia A who are hereditarily deffective in factor VIII:C, the active factor VIII:C is absent in the blood, and hence the blood coagulation function is not effectively exhibited, which results in a bleeding disorder. Therefore, factor VIII:C is supplied to the patient for hemostatic treatment of hemophilia A.

For supply of factor VIII:C, fresh freezed human blood plasma as well as cryoprecipitate, which is a precipitate obtained by thawing the above freezed blood plasma, have been employed, and recently, a factor VIII:C concentrate containing factor VIII:C at a high titer has been employed.

However, though the factor VIII:C concentrate has a high titer, it contains a large amount of impurities such as fibrinogen and fibronectin and further has a risk of infections with viruses such as AIDS virus and hepatitis virus since it is supplied from human blood plasma. In addition, since factor VIII:C is only present in blood plasma in trace amounts, it is very troublesome to obtain a highly purified factor VIII:C.

For these reasons, genetic engineering techniques have recently been used in order to prepare a cheap, safe factor VIII:C preparation which is not derived from human plasma and is free from contamination of viral impurities. These include the cloning of a gene coding for human factor VIII:C and the expression of the gene in cultured animal cells to prepare factor VIII:C.

It was reported that DNA of human factor VIII:C has been cloned [J. Gitschier et al., Nature 312, 326-330 (1984)] and the cDNA has been expressed in hamster kidney cells [W. I. Woods et al., Nature 312, 330-337 (1984)]. Thereafter, it was also reported that cDNA of human factor VIII:C has been cloned and the cDNA has been expressed in mammalian cells [J. J. Toole et al., Nature 312, 342-347 (1984); M. A. Truett et al., DNA 4, 333-349 (1985); A. Pavirani et al., Bio/technology 5, 389-392 (1987)]. However, the genetic expression described in the above literature is transient and the expressed amount of factor VIII:C is quite low since factor VIII:C is a glycoprotein having a high molecular weight, which is not satisfactory for the industrial production of factor VIII:C.

The cDNA of human factor VIII:C comprises a DNA sequence of about 7,000 to 8,000 bases wherein 7053 bases code for factor VIII:C. Human factor VIII:C is a protein comprising 2351 amino acid residues wherein a peptide portion comprising 19 amino acid residues at amino terminus is a signal peptide, which is cleaved by a signal peptidase when passing through membranes to produce the mature factor VIII:C comprising 2332 amino acid resides.

As mentioned above, factor VIII:C is one of a group of in vivo proteins having a very high molecular weight. When such a protein molecule having a high molecular weight is expressed by a genetic recombination technique, there appear various difficulties which are not found in the expression of molecules having a lower molecular weight.

In order to solve such problems, the following method has been attempted where a part of the factor VIII:C gene is modified.

It is believed that factor VIII:C has a domain structure of A1-A2-B-A3-C1-C2 in which A1, A2 and A3 have homologous amino acid sequences, and C1 and C2 have also homologous amino acid sequences [G. A. Vehar et al., Nature 312, 337-342 (1984)]. It is supposed that a part or all of the B domain is unnecessary for the essential cofactor activity of human factor VIII:C. There are several reports on the preparation of modified factor VIII:C, i.e. natural type active human factor VIII:C, for the purpose of producing factor VIII:C species which have a minimized molecular weight but still retain the biological activity in order to increase an expressed amount of the gene. Said natural type active human factor VIII:C means a protein which lacks a part or all of the peptide of the B domain ranging from Ser-741 to Arg-1648 of natural human factor VIII:C, wherein the amino terminus of the light (L) chain is covalently bound by a peptide bond to the carboxy terminus of the heavy (H) chain.

In the present specification, the natural type active human factor VIII:C (i.e. modified factor VIII:C) is abbreviated, for example, as follows.

A modified factor VIII:C wherein a polypeptide fragment (H chain) ranging from Ala-1 to Arg-740 is covalently linked to a polypeptide fragment (L chain) ranging from Glu-1649 to Tyr-2332 is abbreviated as "740 Arg-1649 Glu factor VIII:C" (hereinafter, similar expressions are used). This 740 Arg-1649 Glu factor VIII:C may be referred to as "RE". J. J. Toole et al. describe the expression of 981 Pro-1563 Asp factor VIII:C and 759 Thr-1640 Pro factor VIII:C in COS cells [Proc. N.A.S. USA 83, 5939-5942 (1986)] and in Chinese hamster ovary cells (CHO cells) (PCT application WO86/06101) using an adenovirus major late promoter. D. L. Eaton et al. describe the expression of 796 Gln-1563 Asn factor VIII:C in COS cells using an SV 40 early promoter [Biochemistry 25, 8343-8347 (1986)].

Although such modified factor VIII:C genes, wherein a region coding for the B domain has been deleted, can increase an expressed amount of factor VIII:C, it is still insufficient for the production of factor VIII:C on an industrial scale. Under such circumstances, there has been desired to develop a method for the production of active factor VIII:C which can greatly increase the yield of factor VIII:C.

Thus, the technical problem underlying the present invention is to provide a novel system for the expression of a protein having the biological activity of human factor VIII:C in animal cells at a higher degree. The solution is achieved by expressing human factor VIII:C or a derivative thereof under the control of the chicken β-actin promoter. In accordance with the present invention, human factor VIII:C can be expressed at an extremely higher degree as compared with the conventional procedures employing other promoters and host cells.

That is, the present invention provides a novel expression system for animal cells using a chicken β-actin promoter for the production of human factor VIII:C in genetic recombination techniques as well as a process for preparing human factor VIII:C which can be utilized in an industrial scale.

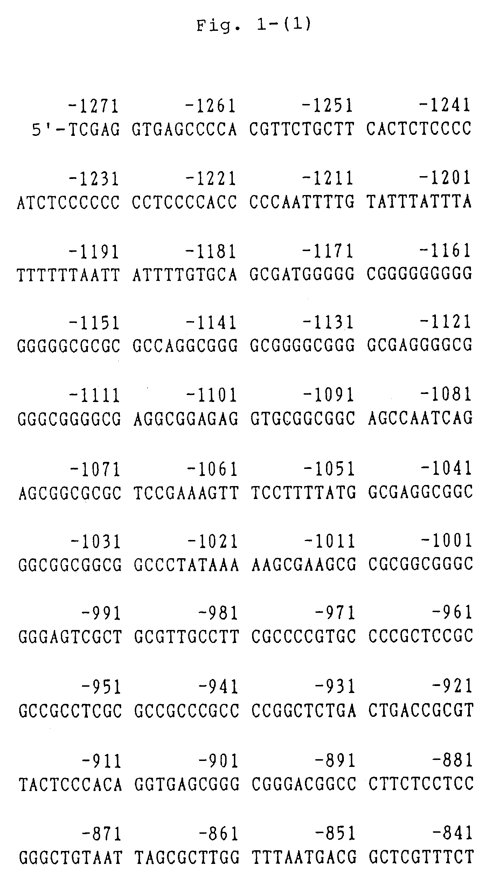

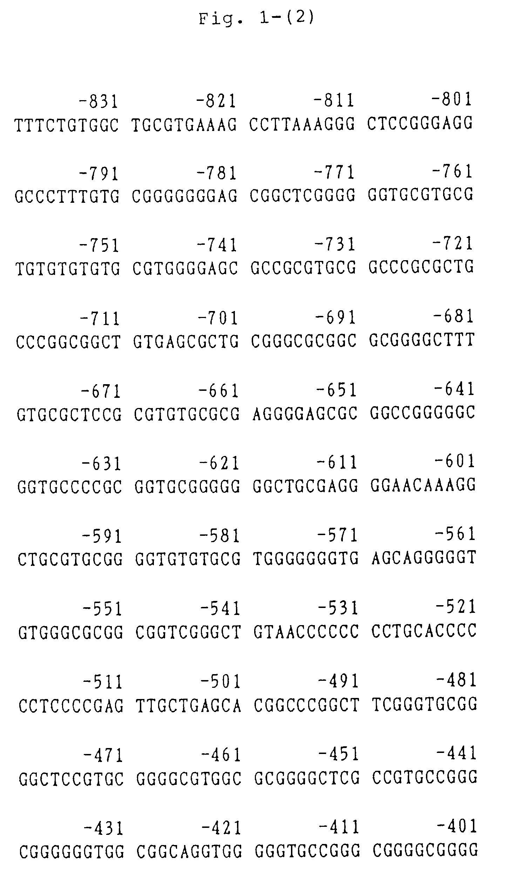

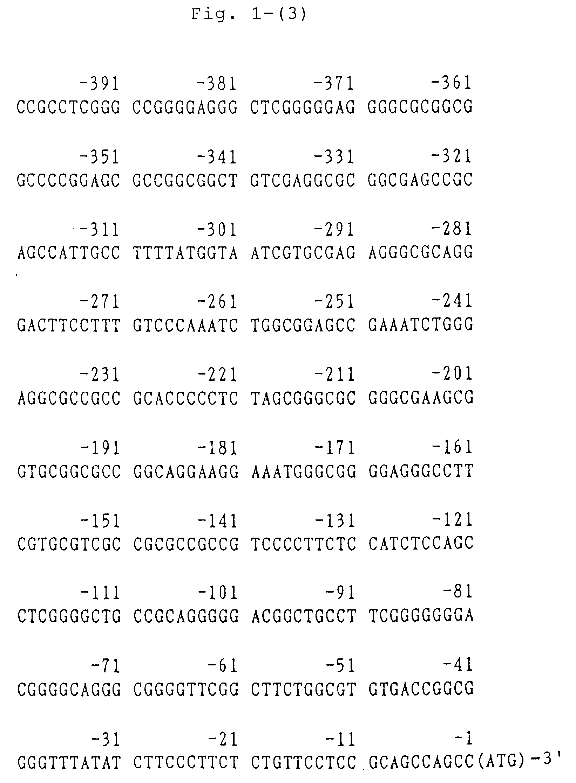

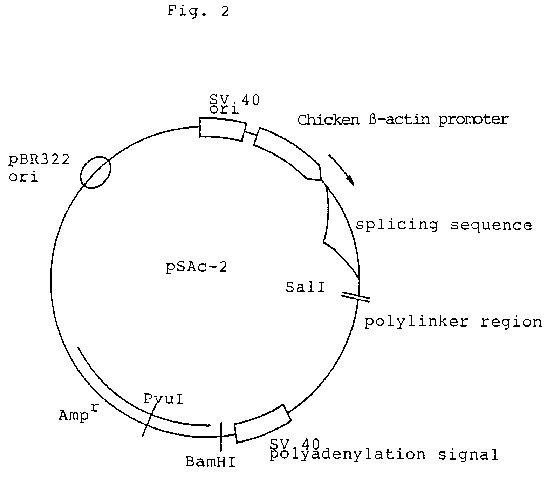

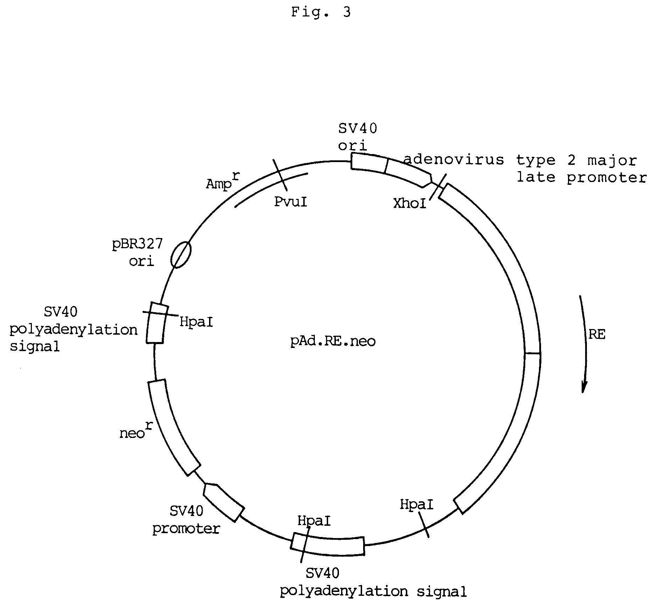

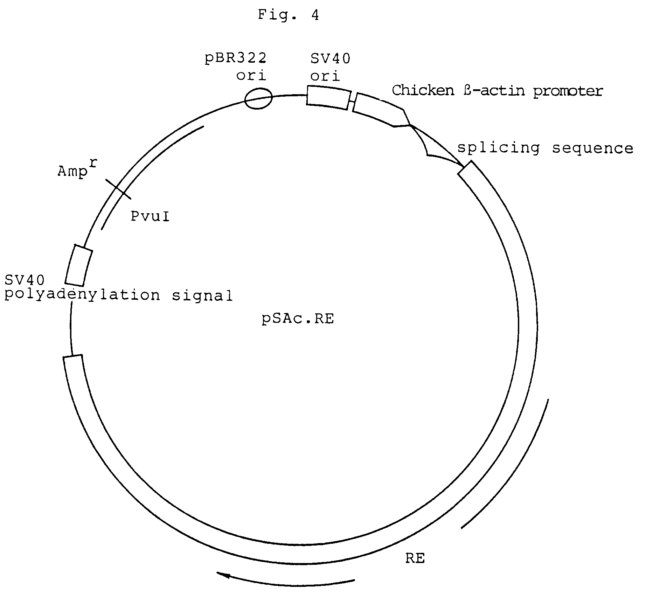

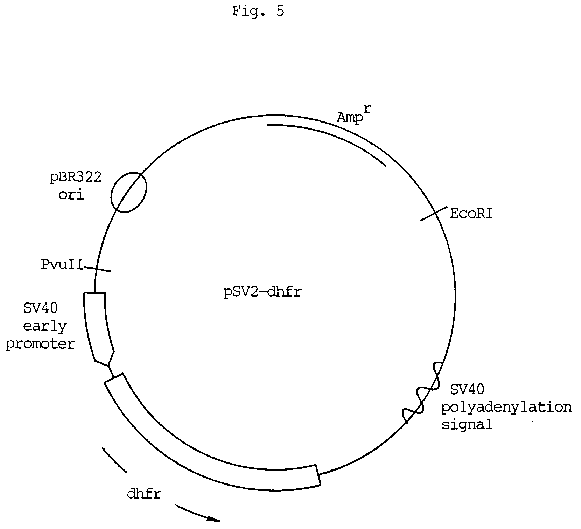

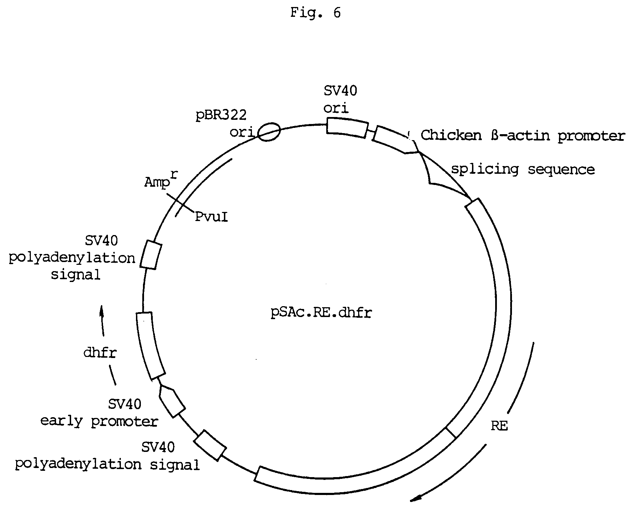

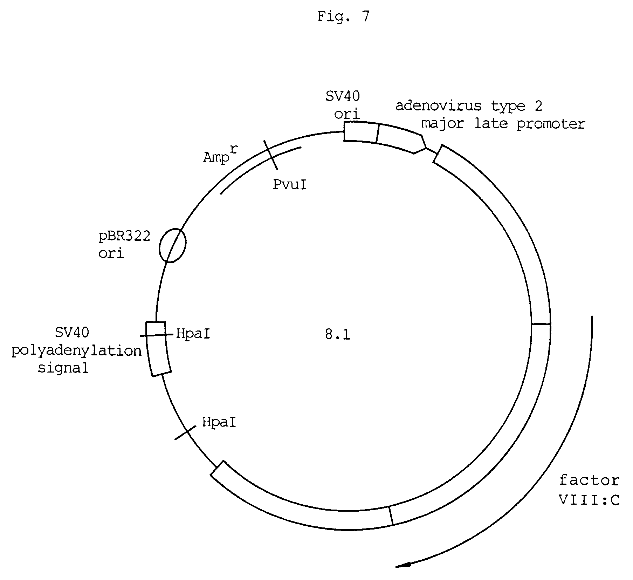

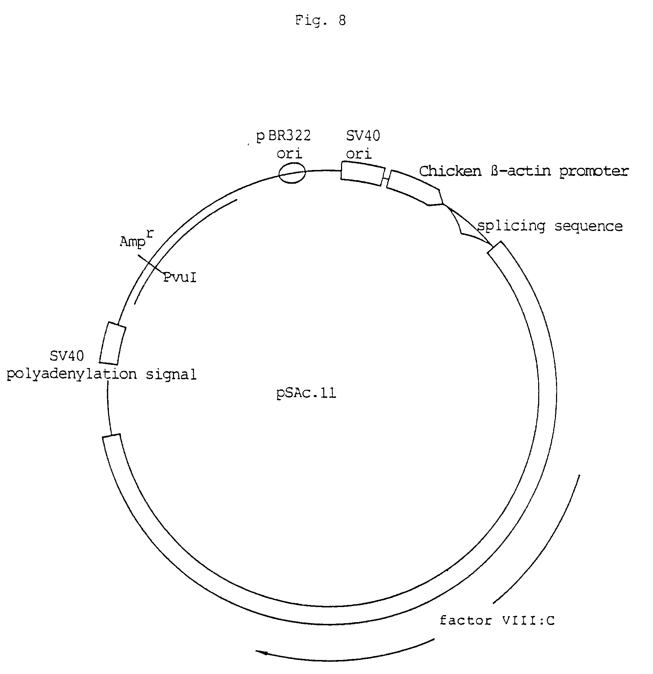

Fig. 1 shows the DNA sequence of a chicken β-actin promoter; Fig. 2 shows plasmid pSAc-2 which is an expression plasmid containing the chicken β-actin promoter Fig. 3 shows the RE expression vector pAd.RE.neo which is a plasmid containing an adenovirus-2 major late promoter; Fig. 4 shows the RE expression vector pSAc.RE; Fig. 5 shows the dihydrofolate reductase (DHFR) expression vector pSV2-dhfr; Fig. 6 shows the expression vector pSAc.RE.dhfr; Fig. 7 shows the full-length factor VIII:C expression vector; and Fig. 8 shows the full-length factor VIII:C expression vector pSAc.11.

The process of the present invention employs a chicken β-actin promoter, which is a gene fragment having the DNA sequence of Fig. 1.

β-Actin is one of the major structural proteins of eucaryotes from protozoa to human beings. It is present in all cells and is associated with a variety of cellular functions. As a similar genetic recombination technique to the technique of the present invention employing β-actin promoter, there has been reported an expression system for an exogenous gene with use of a human β-actin promoter [P. Gunning et al., Proc. N.A.S. USA 84, 4831-4835 (1987) and Japanese Patent First Publication (Kokai) No. 262995/1987]. According to this technique, the expression efficiency is improved as compared with the conventional SV 40 promoter, but the promoter of this technique is only about 1.7 times more active than the SV 40 early promoter

This is not sufficient for an industrial production.

A Comparison of the structural genes of chicken β-actin used in the present invention and human β-actin indicates a difference of only 2 amino acid residues among the 367 encoded amino acid residues but only a 50.7 % homology of the DNA sequences of to the promoter region.

According to a preferred embodiment of the present invention, there is used as a promoter a DNA fragment containing 1271 base pairs starting from position -1275 thymine (T) to -5 cytosine (C) in the sequence shown in Fig. 1. The desired factor VIII:C can be extremely effectively expressed with this promoter. It is generally known that the promoter activity is little affected by a deletion of some 10 to 30 base pairs upstream (5′ end) from the initiation codon (ATG). In case of the chicken β-actin promoter of the present invention, however, it has been found by the present inventors that the 3′ end of the promoter should preferably contain the DNA sequence up to the cytosine (C) five base pairs upstream of the initiation codon (ATG) of the original structural gene of β-actin. The promoter activity is deteriorated if nucleotides upstream of the cytosine (C) -5 of Fig. 1 are deleted. This is due to the fact that these DNA sequences participate in a splicing after the transcription of messenger RNA.

The factor VIII:C gene to be incorporated downstream of chicken β-actin promoter in the expression vector is a gene coding for the above-mentioned factor VIII:C molecule comprising 2332 amino acids. In a preferred embodiment, there is used the above-mentioned factor VIII:C gene wherein a gene region coding for domain B of factor VIII:C has been partly or wholly deleted, because the incorporation of the factor VIII:C further improves an expression efficiency.

In the present invention, human factor VIII:C comprising 2332 amino acids is referred to as "full-length factor VIII:C" while human factor VIII:C with deletion of a part or whole of the B domain is called "modified factor VIII:C".

One of the preferred factor VIII:C genes used in the present invention is a gene wherein a DNA sequence coding for the natural signal peptide of factor VIII:C is linked to a DNA sequence coding for a protein which is prepared by directly linking Arg-740 contained in the H chain of human factor VIII:C to Glu-1649 contained in the L chain of human factor VIII:C, i.e. to a DNA sequence coding for a modified factor VIII:C [= 740 Arg-1649 Glu factor VIII:C (abbreviated as "RE").The plasmid wherein the gene coding for the RE is incorporated has been deposited with the ATCC as transformant E. coli HB101(RE) under the accession number 53517.

The human factor VIII:C expression vector of the present invention is preferably constructed by combining (a) a DNA sequence wherein a DNA sequence coding for a polyadenylation signal is linked to the 3′ end of a gene coding for a full-length factor VIII:C or a modified factor VIII:C, containing a DNA sequence coding for the signal peptide necessary for secretion; (b) a DNA sequence comprising an origin of replication for E. coli and a drug resistance gene; (c) an origin of replication (ori) which functions in eucaryotic cells; and (d) the above chicken β-actin promoter. For example, a gene coding for the human factor VIII:C can be incorporated downstream of the chicken β-actin promoter into the vector (e.g. pSAc-2 (Fig. 2)), by which the desired human factor VIII:C can be produced in large amounts.

As a host cell for the introduction of the abovementioned human factor VIII:C expression vector, any animal cell can be employed as far as it is capable of expressing the desired human factor VIII:C. Animal cells are preferred in which the desired transformants can be easily separated, such as Chinese hamster ovary cell (CHO cell).

The transformation of the host cells can be effected by known methods, including for example the calcium phosphate method [Virology 52, 456 (1973)],the DEAE-DEXTRAN method [DNA cloning vol. II IRL press, Oxford, 143-190 (1985)],or the electroporation method [Pro. N.A.S. 81, 7161-7165 (1984)]. For facilitating the selection of a transformant, animal cells deficient in a marker gene are employed. For example, a CHO cell deficient in a dihydrofolate reductase (DHFR) gene is subjected to co-transfection [Cell 16, 777-785 (1979)] with the factor VIII:C expression vector together with a DHFR expression vector such as pSV2-dhfr [Mol. Cell. Biol. 1, 854-864 (1981)], and screened in a selection medium deficient in a nucleoside. As a result a CHO cell is obtained in which the DHFR gene is introduced and expressed and which might further contain the human factor VIII:C gene. In addition, a possibility of selection and separation of a CHO cell where both genes are introduced further increases when the DHFR expression vector is employed in a smaller amount than the factor VIII:C expression vector in the above co-transfection (e.g. the DHFR expression vector is employed in an amount of 1/50 of that of the factor VIII:C expression vector). More preferably, a factor VIII:C-DHFR coexpression vector wherein a DHFR gene was previously incorporated into the factor VIII:C expression vector is introduced into the host cell so that both the factor VIII:C gene and the DHFR gene are simultaneously incorporated into the chromosomal DNA of the host cell more effectively and adjacent to each other. Further, the thus obtained transformant can exhibit an amplification effect of the factor VIII:C gene as mentioned hereinbelow, and consequently, can produce a large amount of factor VIII:C.

In case that an amplifiable gene such as DHFR is employed, the factor VIII:C gene, which is incorporated into the host together with the DHFR gene, can be simultaneously amplified by culturing the host cell under a condition suitable for gene amplification, e.g. in the presence of methotrexate in case of the DHFR gene. As a result, factor VIII:C is produced in a greatly increased amount per cell and is secreted into the culture in a large amount.

According to the present invention, the factor VIII:C-producing transformant cell can also be cultured with a serum-free medium which can produce factor VIII:C in a large amount more effectively than with the usual serum medium.

In addition, various additives may be added to the culture medium during the culture of the transformant so that the efficiency of the factor VIII:C production by the transformant is extremely increased. Such additives include proteins derived from living bodies such as albumin and von Willebrand factor, which are serum proteins. Preferable examples of the additives further include polyethylene glycol, sodium selenite, cyclodextrin, a protease inhibitor such as ε-aminocaproic acid, aprotinin or PMSF (phenylmethanesulfonyl fluoride).

The factor VIII:C protein produced and accumulated in the culture can be separated and purified from the culture supernatant by a combination of the known separation procedures after the cells are removed and the culture solution is concentrated.

Such known separation and purification procedures include, for example, a method utilizing a difference in solubility (e.g. salting-out or precipitation in solvent), a method utilizing a difference in molecular weight (e.g. dialysis, ultrafiltration, gel filtration or SDS-polyacrylamide gel electrophoresis), a method utilizing a difference in an electric charge (e.g. ion exchange chromatography), a method utilizing specific affinity (e.g. affinity chromatography), a method utilizing a difference in hydrophobicity (e.g. reverse phase high performance liquid chromatography) or a method utilizing a difference in isoelectric point (e.g. isoelectric electrophoresis).

The solution containing the factor VIII:C protein obtained by the present invention may optionally be lyophilized to give a powdery product. The lyophilization may be carried out in the presence of a stabilizing agent such as sorbitol, mannitol, dextrose, an amino acid, maltose, glycerol or human serum albumin (HSA).

According to the process of the present invention, the production efficiency of factor VIII:C can greatly be increased, while it was very difficult to highly express factor VIII:C in the conventional genetic recombination procedure. The process for preparing factor VIII:C of the present invention utilizing the chicken β-actin promoter provides a technique to produce factor VIII:C at an efficiency at least ten times higher than the expression system utilizing an adenovirus type 2 major late promoter which is known to be one of the most powerful promoters, as shown in the Examples described hereinbelow. It has not been known that the chicken β-actin promoter is quite effective in the expression of factor VIII:C, which has hitherto been considered to be extremely difficult as mentioned hereinabove.

The present invention is more specifically illustrated by the following Preparation and Examples but should not be construed to be limited thereto.

### Preparation

### (1) Preparation of a promoter region of a chicken β-actin gene promoter:

A plasmid pAZ1037 which contains a first exon, a first intron and a part of a second exon of a chicken β-actin gene promoter and a CAT gene linked thereto [Nature 314, 286-289 (1985)] was digested with restriction enzyme NcoI (NEB #193). In the present invention, the following procedures were carried out so that the splicing region between the first intron and the second exon of the chicken β-actin gene promoter correctly functions when used as the expression vector.

That is, the NcoI-digested DNA was treated with S1 nuclease (Takara #2410A) to delete the 5′ overhanging region and only one base pair adjacent thereto. In this reaction, a sample DNA (10 µg) was treated with 150 units of S1 nuclease in a solution (80 µl) of 30 mM sodium acetate, pH 4.6, 100 mM NaCl, 1 mM ZnSO₄ at 37°C for 1 to 4 minutes.

After treating the DNA with S1 nuclease, the treated DNA was further treated with T4 DNA polymerase (Takara #2040A) to modify the single strand moiety of the DNA and thereto was linked a synthesized DNA pCCAAGCTTGG 5′ end of which is phosphorylated (pHindIII linker, NEB #1050) with T4 DNA ligase (Takara #2011A) to cyclize. E. coli HB101 strain was transformed with the obtained DNA solution. After separating a single colony, a plasmid DNA in the transformed cells was collected and digested with restriction enzymes HindIII (NEB #104) and NarI (NEB #191). 6% Acrylamide gel electrophoresis was conducted and a clone was selected which contained a DNA fragment with a suitable size. The DNA fragment was cloned into the SalIKpnI site of phage vector M13mp19 (NEB #400-19) and a DNA sequence about the HindIII site was determined by a dideoxy method [Proc. N.A.S. 74, 5463-5477 (1977)] to screen the desired clone.

The thus obtained plasmid clone p28 retained a DNA sequence ranging from the splicing region to the structural gene of the natural chicken β-actin gene and had a structure where a gene at 3′ end from the initiation codon (ATG) was deleted and thereto HindIII site was incorporated.

### (2) Construction of expression vector, pAc-2:

A plasmid pSV2-cat containing a splicing region for SV 40 early transcription and a polyadenylation signal [Molecular Cell Biology 2, 1044-1051 (1982)] was digested with restriction enzyme MflI (Takara #1070A). The cleaved sites were modified with T4 DNA polymerase and thereto was linked a phosphorylated HindIII linker with T4 DNA ligase. The obtained plasmid was further digested with restriction enzymes HindIII and BamHI (NEB #136) and the cleaved fragments were subjected to 6 % acrylamide gel electrophoresis to extract a HindIII-BamHI fragment of about 900 bp. The plasmid p28 constructed in the above preparation (1) was digested with restriction enzymes HindIII and BamHI and dephosphorylated with alkaline phosphatase derived from calf intestine (Takara #2250A). This was linked to the above HindIII-BamHI fragment of about 900 bp with T4 DNA ligase to cyclize to give a plasmid pAc-2.

### (3) Construction of an expression vector, pSAc-2:

The same plasmid pSV2-cat as used in the above preparation (2) was digested with restriction enzymes AccI (NEB #161) and SphI (NEB #182) and the cleaved sites were blunt-ended with T4 DNA polymerase. This was linked and cyclized in the presence of a phosphorylated XbaI linker (NEB #1032) with T4 DNA ligase to prepare a plasmid pSV-cat-delE [Proc. Natl. Acad. Sci. USA 83, 9537-9541 (1986)].

This plasmid pSV-cat-delE was digested with restriction enzyme HindIII and the cleaved sites were modified with T4 DNA polymerase. This was linked and cyclized in the presence of a phosphorylated XhoI linker NEB #1030) with T4 DNA ligase. The obtained plasmid was further digested with restriction enzymes EcoRI (NEB #101) and XhoI (NEB #146) and the cleaved fragments were subjected to 1 % agarose gel electrophoresis to extract an EcoRI-XhoI fragment of about 2 kbp. The plasmid pAc-2 constructed above was digested with restriction enzymes XhoI and EcoRI to give an XhoI-EcoRI fragment of about 2.2 kbp, to which was linked the above EcoRI-XhoI fragment of about 2 kbp to cyclize to prepare a plasmid pSAc-2 (Fig. 2).

### Example 1 (Construction of modified human factor VIII:C expression vector pSAc.RE)

RE expression vector pAd.RE.neo (Fig. 3) containing an adenovirus type 2 major late promoter was digested with the restriction enzyme HpaI and thereto was inserted phosphorylated XhoI linker with T4 DNA ligase to construct a cyclized PAd.RE.3X.

The obtained pAd.RE.3X was digested with the restriction enzyme XhoI and then subjected to an agarose gel electrophoresis. A DNA fragment of about 4.7 kbp containing RE cDNA was extracted from the gel.

On the other hand, the expression vector pSAc-2 (Fig. 2) containing chicken β-actin promoter was digested with the restriction enzyme SalI and then dephosphorylated with alkaline phosphatase derived from calf intestine.

This DNA and the above RE cDNA fragment were ligated to each other to cyclize with T4 DNA ligase to give an RE expression vector pSAc.RE containing chicken β-actin promoter (Fig. 4).

### Example 2 (Expression of RE in COS cell)

COS-1 cells were placed on a 6-well multiplate for tissue culture (manufactured by Falcon) at a cell number of each 2 x 10⁵ cells/well and to the plate was added Dulbecco's Modified Eagle medium supplemented with 10 % fetal calf serum (3 ml), which was incubated in CO₂ incubator at 37°C overnight.

Next day, the COS cells were transformed with the RE expression vector pSAc.RE by a DEAE-dextran method by the following procedure. The COS cells were also transformed with an RE expression vector pAd.RE.neo containing adenovirus type 2 major late promoter (hereinafter referred to as "Ad2-MLP") in place of the pSAc.RE as a control by the same procedure.

A solution (0.9 % NaCl, 50 µl) of DEAE-dextran (2 mg/ml) from transfection kit (CellPhect Trasfection Kit #17-0595-01 manufactured by Pharmacia) was diluted two-fold with 0.05 M Tris-HCl buffer (pH 7.5) and thereto was added 0.05 M Tris-HCl buffer (pH 7.5) (100 µl) containing pSAc.RE (2 µg).

The cells in each well were washed twice with a PBS(-) solution and then the above mixture of DNA and DEAE-dextran (200 µl) was added dropwise to the cells in each well. After standing at room temperature for 15 minutes, the solution was removed from each well and the cells were washed twice with 0.05 M Tris-HCl buffer (pH 7.5). Dulbecco's Modified Eagle medium (MEM) supplemented with 10 % fetal calf serum (3 ml) was added thereto, and the mixture was incubated in CO₂ incubator.

Five days to eight days after the transformation, a factor VIII:C activity of the culture supernatant was measured with Coatest® factor VIII:C kit (manufactured by Kabi Vitrum). The results are shown in Table 1. In all Examples hereinafter, the measurement of the factor VIII:C activity was carried out by using this kit.

**Table 1**

| Vector | Promoter | Factor VIII:C Activity (mU/ml) | |
|---|---|---|---|
| | | 5 days after | 8 days after |
| pSAc.RE. | Chicken β-Actin | 14 | 45 |
| pAd.RE.neo | Ad2-MLP | 3 | 5 |

### Example 3 (Co-transfection of CHO (DHFR⁻) cells with RE expression vector and DHFR expression vector)

Dihydrofolate reductase defective (DHFR-) cell line of Chinese hamster ovary (CHO) cells, DG44 [Somatic Cell and Molecular Genetics 12, 555-566 (1986)] (2 x 10⁵ cells/well) was incubated on 6-well multiplate for tissue culture (manufactured by Falcon) with MEM alpha medium (containing nucleoside) supplemented with 10 % fetal calf serum (3 ml/well) in CO₂ incubator at 37°C overnight.

The used RE expression vector pSAc.RE and the DHFR expression vector pSV2-dhfr (Fig. 5) were linearized by digestion with the restriction enzyme PvuI.

The CHO cells DG44 were co-transfected with the above RE expression vector and the DHFR expression vector by calcium phosphate method. That is, the above linearized pSAc.RE (10 µg) and pSV2-dhfr (200 ng) were mixed with a carrier DNA (calf thymus DNA was used herein) (10 µg) in 0.25 M CaCl₂ (100 µl) and thereto was added dropwise HEPES buffered saline (280 mM NaCl, 1.5 mM Na₂HPO₄, 50 mM HEPES, pH 7.1) (100 µl). The mixture was stirred and the formed DNA-calcium phosphate complex was added dropwise to a plate where the CHO cells DG44 were cultured as mentioned above to transfect the CHO cells. As a control, pAd.RE.neo was also linearized by digestion with the restriction enzyme PvuI and introduced into the CHO cells together with pSV2-dhfr in the same manner as pSAc.RE.

After culturing for 15 hours, the culture medium was replaced with a nucleoside-defective MEM alpha medium supplemented with 10 % fetal calf serum (hereinafter referred to as "selection medium") (3 ml/well). Thereafter, the culture was continued while replacing the medium with a fresh selection medium every 3 to 4 days. As a result, only DHFR(+) cells were survived where pSV2-dhfr was introduced. After co-transfection and culturing for three weeks in the selection medium, factor VIII:C activity was measured for the culture supernatant 4 days after replacement with a fresh medium. The results are shown in Table 2.

**Table 2**

| Vector | Promoter | Factor VIII:C activity (mU/ml) |
|---|---|---|
| pSAc.RE. | Chicken β-Actin | 507 |
| pAd.RE.neo | Ad2-MLP | 1.4 |

### Example 4 (Cloning of RE-producing CHO transformant and gene amplification)

The RE-producing CHO cell, CHO.ARE44, obtained in Example 3 was cloned by a limiting dilution using 96 well multiplate.

The cloned cells were placed on a 24-well multiplate for tissue culture (manufactured by Nunc CO., Ltd.) and were cultured with a selection medium (1 ml/well). After the cells became confluent, a factor VIII:C activity of the culture supernatant was measured in each clone. Table 3 shows results for typical clones.

For gene amplification, each clone was cultured in a selection medium containing 10 nM methotrexate (hereinafter referred to as "MTX"). The culture was continued for two weeks while replacing the culture medium with a fresh selection medium containing 10 nM MTX every 3 to 4 days, and thereafter, a factor VIII:C activity of the culture supernatant was measured. The results are shown in Table 3.

**Table 3**

| Clone | Factor VIII:C activity (mU/day/10⁶ cells) | |
|---|---|---|
| | Activity after cloning | After culture with addtion of 10 nM MTX |
| ARE44-1 | 210 | 500 |
| ARE44-3 | 266 | 1180 |
| ARE44-4 | 352 | 1300 |

### Example 5 (Construction of expression vector wherein RE gene and DHFR gene are ligated on the same plasmid)

The expression vector pSAc-2 containing chicken β-actin promoter was digested with the restriction enzyme BamHI and the cleaved sites were blunt-ended with T4 DNA polymerase in the presence of dXTP.

On the other hand, the DHFR expression vector pSV2-dhfr was digested with the restriction enzymes PvuII and EcoRI and the cleaved sites were blunt-ended with T4 DNA polymerase in the same manner as pSAc-2. Agarose gel electrophoresis was carried out and the DNA fragments containing DHFR gene were extracted from the gel.

The blunt end of this DNA fragment was ligated to the above blunt-ended linear pSAc-2 with T4 DNA ligase to cyclize and construct plasmid pSAc.dhfr.

The plasmid pSAc.dhfr was digested with the restriction enzyme SalI and then dephosphorylated in the same manner as in Example 1 and thereto RE cDNA was ligated to cyclize. In this way, an expression vector pSAc.RE.dhfr (Fig. 6) was constructed where both RE and DHFR genes were ligated on the same plasmid.

### Example 6 (Transformation of CHO cell DG44 with RE expression vector pSAc.RE.dhfr)

The CHO cells DG44 were cultured on a 6-well multiplate in the same manner as in Example 3. The expression vector pSAc.RE.dhfr used for transformation was previously linearized by digestion with the restriction enzyme PvuI.

As a control, the expression vector pAd.RE.dhfr, wherein the chicken β-actin in pSAc.RE.dhfr was replaced with the adenovirus type 2 major late promoter, was also linearized by PvuI digestion.

Using each 10 µg of these DNAs, the CHO cells DG44 were transformed by a calcium phosphate method in the same manner as described in Example 3. Sixteen hours after the culture, the culture medium was replaced with a selection medium (3 ml) and the culture was continued for two weeks while replacing the medium with a fresh selection medium every 3 to 4 days, and a factor VIII:C activity of the culture supernatant was measured. The results are shown in Table 4.

**Table 4**

| Vector | Promoter | Factor VIII:C activity (mU/ml) |
|---|---|---|
| pSAc.RE.dhfr | Chicken β-Actin | 300 |
| pAd.RE.dhfr | Ad2-MLP | 32 |

### Example 7 (Construction of full-length factor VIII:C expression vector pSAc.11)

A full-length factor VIII:C expression vector 8.1 containing an adenovirus type 2 major late promoter (Fig. 7) (US 87/0814) was digested with the restriction enzyme HpaI and thereto was inserted a phosphorylated XhoI linker with T4 DNA ligase to cyclize and construct a plasmid 8.1.3X.

The obtained plasmid 8.1.3X was digested with the restriction enzyme XhoI, subjected to an agarose gel electrophoresis and then a DNA fragment of about 7.5 kbp containing factor VIII:C cDNA was extracted from the gel.

On the other hand, the expression vector pSAc-2 containing chicken β-actin promoter (Fig. 2) was digested with the restriction enzyme SalI and dephosphorylated with alkaline phosphatase derived from calf intestine.

This linearized vector and the above-mentioned DNA fragment containing factor VIII:C cDNA of about 7.5 kbp were ligated to each other with T4 DNA ligase to cyclize and construct a full-length factor VIII:C expression vector pSAc.11 (Fig. 8).

### Example 8 (Expression of full-length factor VIII:C in COS cells)

The COS-1 cells were transformed with the full-length factor VIII:C expression vector pSAc.11 containing the chicken β-actin promoter by a DEAE-dextran method in the same manner as in Example 2 in order to study an expression efficiency of the expression vector introduced in COS cells. As a control, the same experiment was carried out with the full-length factor VIII:C expression vector 8.1 where the chicken β-actin promoter in pSAc.11 was replaced with an adenovirus type 2 major late promoter. A factor VIII:C activity of the culture supernatant was measured after transformation. The results are shown in Table 5.

**Table 5**

| Vector | Promoter | Factor VIII:C acitivity (mU/ml) | |
|---|---|---|---|
| | | after 5 days | after 8 days |
| pSAc.11 | Chicken β-actin | 0.6 | 2 |
| 8.1 | Ad2-MLP | 0 | 0 |

### Example 9 (Co-transfection of CHO cells with full-length factor VIII:C expression vector and DHFR expression vector)

Following the same manner as in the case of the co-transfection of CHO cells described in Example 3, the co-transfection was conducted with the full-length factor VIII:C expression vector pSAc.11 and the DHFR expression vector pSV2-dhfr.

The used vectors pSAc.11 and pSV2-dhfr have been digested with the restriction enzyme PvuI.

As a control, the same experiment was carried out with the full-length factor VIII:C expression vector plasmid 8.1 containing the adenovirus type 2 major late promoter instead of pSAc.11 digested with the restriction enzyme PvuI.

After co-transfection and culturing for three weeks in the selection medium, a factor VIII:C activity was measured for the culture supernatant four days after replacement with a fresh medium. The results are shown in Table 6.

**Table 6**

| Vector | Promoter | Factor VIII:C activity (mU/ml) |
|---|---|---|
| pSAc.11 | Chicken β-Actin | 50 |
| 8.1 | Ad2-MLP | 0 |

### Example 10 (Cloning of full-length factor VIII:C-producing CHO transformant and gene amplification)

The full-length factor VIII:C-producing CHO transformant obtained in Example 9 was cloned by a limiting dilution method. The cloned cells were placed on a 24-well multiplate for tissue culture (manufactured by Nunc Co., Ltd.) and cultured with a selection medium (1 ml/well). When the cells became confluent, a factor VIII:C activity of the culture supernatant was measured for each clone. Table 7 shows results obtained in typical clones.

Each clone was also cultured with a selection medium containing 10 nM MTX. The culture was continued while replacing the medium with a selection medium containing 10 nM MTX every 3 to 4 days and a factor VIII:C activity of the culture supernatant was measured. The results are shown in Table 7.

**Table 7**

| Clone | Factor VIII:C activity (mU/day/10⁶ cells) | |
|---|---|---|
| | Activity after cloning | After addition of 10 nm MTX |
| 1 | 48 | 168 |
| 2 | 59 | 188 |
| 3 | 49 | 212 |
| 4 | 66 | 206 |
| 5 | 73 | 197 |

### Example 11 (Culture of factor VIII:C-producing CHO transformant with serum-free medium and effect of additives)

The RE-producing CHO cells ARE.44 clones 3 and 4 obtained in Example 4 after gene amplification were placed on a 6-well multiplate for tissue culture (manufactured by Falcon) at 5 x 10⁵ cells/well and cultured with a selection medium (serum medium) (3 ml/well) or serum-free medium (manufactured by Ajinomoto Co., Ltd., ASF medium 104), supplemented with 1 % human serum albumin, both medium being supplemented with 10 nM MTX, for 3 to 4 days. Thereafter, each medium was replaced with a fresh medium and a factor VIII:C activity of the culture supernatant was measured 24 hours after the culture. The results are shown in Table 8.

**Table 8**

| Clone | Factor VIII:C activity (mU/day/10⁶ cells) | |
|---|---|---|
| | Serum medium | ASF 104 + 1 % HSA |
| ARE44-3 | 500 | 2400 |
| ARE44-4 | 600 | 4900 |

## Claims (Claims for the following Contracting State(s): AT, BE, CH, DE, FR, GB, IT, LI, LU, NL, SE)

1. A plasmid for the expression of a protein having the biological activity of human factor VIII:C which comprises
(a) a chicken β-actin promoter or a derivative thereof excluding the human β-actin promoter and
(b) downstream of the promoter (a) a gene coding for said protein having the biological activity of human factor VIII:C.

2. The plasmid of claim 1, wherein said chicken β-actin promoter contains the complete DNA sequence shown in Fig. 1 or a part thereof having a promoter activity.

3. The plasmid according to claim 1, wherein the derivative of the chicken β-actin promoter is a DNA fragment of the chicken β-actin promoter containing at its 3'-end at least the nucleotides up to the C at position -5 calculated from the initiation codon ATG of the original structural β-actin gene.

4. The plasmid according to any one of claims 1 to 3 which additionally comprises at least one origin of replication (ori).

5. The plasmid according to claim 4, wherein the ori is derived from SV40.

6. The plasmid according to any one of claims 1 to 5, wherein a DNA sequence encoding a signal peptide is linked to the gene encoding the protein having the biological activity of human factor VIII:C.

7. The plasmid according to any one of claims 1 to 6, wherein the protein having the biological activity of human factor VIII:C is a protein in which the Arg-residue at position 740 at the amino terminus of the H chain of human factor VIII:C is covalently linked to the Glu-residue at position 1649 at the carboxy terminus of the L chain of human factor VIII:C.

8. The plasmid according to any one of claims 1 to 7 which additionally comprises an amplifiable gene.

9. A microorganism which is transformed with an expression plasmid according to any one of claims 1 to 8.

10. A microorganism according to claim 9 which is an animal cell, preferably a Chinese hamster ovary (CHO) cell.

11. A process for the production of a protein having the biological activity of human factor VIII:C which comprises culturing a transformed microorganism according to claim 9 or 10 under suitable conditions in a medium and recovering said protein from the culture.

## Claims (Claims for the following Contracting State(s): ES)

1. A method for the construction of a plasmid for the expression of a protein having the biological activity of human factor VIII:C which comprises incorporating a gene coding for a protein having the biological activity of a human factor VIII:C downstream of a chicken β-actin promoter or a derivative thereof excluding the human β-actin promoter in a vector.

2. The method of claim 1, wherein said chicken β-actin promoter contains the complete DNA sequence shown in Fig. 1 or a part thereof having a promoter activity.

3. The method according to claim 1, wherein the derivative of the chicken β-actin promoter is a DNA fragment of the chicken β-actin promoter containing at its 3'-end at least the nucleotides up to the C at position -5 calculated from the initiation codon ATG of the original structural β-actin gene.

4. The method according to any one of claims 1 to 3, wherein the vector additionally comprises at least one origin of replication (ori).

5. The method according to claim 4, wherein the ori is derived from SV40.

6. The method according to any one of claims 1 to 5, wherein a DNA sequence encoding a signal peptide is linked to the gene encoding the protein having the biological activity of human factor VIII:C.

7. The method according to any one of claims 1 to 6, wherein the protein having the biological activity of human factor VIII:C is a protein in which the Arg-residue at position 740 at the amino terminus of the H-chain of human factor VIII:C is covalently linked to the Glu-residue at position 1649 at the carboxy terminus of the L chain of human factor VIII:C.

8. The method according to any one of claims 1 to 7, wherein the vector additionally comprises an amplifiable gene.

9. A microorganism which is transformed with an expression plasmid obtainable by any one of the methods of claims 1 to 8.

10. A microorganism according to claim 9 which is an animal cell, preferably a Chinese hamster ovary (CHO) cell.

11. A process for the production of a protein having the biological activity of human factor VIII:C which comprises culturing a transformed microorganism according to claim 9 or 10 under suitable conditions in a medium and recovering said protein from the culture.

## Patentansprüche (Patentansprüche für folgende(n) Vertragsstaat(en): AT, BE, CH, DE, FR, GB, IT, LI, LU, NL, SE)

1. Plasmid zur Expression eines Proteins mit der biologischen Aktivität von menschlichem Faktor VIII:C, umfassend
(a) einen Hühner-β-Actinpromotor oder ein Derivat davon, mit Ausnahme des menschlichen β-Actinpromotors, und
(b) stromabwärts des Promotors (a) ein Gen, das das Protein mit der biologischen Aktivität von menschlichem Faktor VIII:C codiert.

2. Plasmid nach Anspruch 1, wobei der Hühner-β-Actinpromotor die vollständige in Figur 1 gezeigte DNA-Sequenz oder einen Teil davon mit einer Promotoraktivität enthält.

3. Plasmid nach Anspruch 1, wobei das Derivat des Hühner-β-Actinpromotors ein DNA-Fragment des Hühner-β-Actinpromotors ist, das an seinem 3'-Ende mindestens die Nucleotide bis zum C an der Position -5 enthält, berechnet von dem Initiationscodon ATG des ursprünglichen β-Actinstrukturgens.

4. Plasmid nach einem der Ansprüche 1 bis 3, das außerdem mindestens einen Replikationsursprung (ori) umfaßt.

5. Plasmid nach Anspruch 4, wobei der ori von SV40 stammt.

6. Plasmid nach einem der Ansprüche 1 bis 5, wobei eine ein Signalpeptid codierende DNA-Sequenz an das das Protein mit der biologischen Aktivität von menschlichem Faktor VIII:C codierende Gen geknüpft ist.

7. Plasmid nach einem der Ansprüche 1 bis 6, wobei das Protein mit der biologischen Aktivität von menschlichem Faktor VIII:C ein Protein ist, bei dem der Arg-Rest an Position 740 am Aminoterminus der H-Kette von menschlichem Faktor VIII:C mit dem Glu-Rest an Position 1649 am Carboxyterminus der L-Kette von menschlichem Faktor VIII:C kovalent verknüpft ist.

8. Plasmid nach einem der Ansprüche 1 bis 7, das außerdem ein amplifizierbares Gen umfaßt.

9. Mikroorganismus, der mit einem Expressionsplasmid nach einem der Ansprüche 1 bis 8 transformiert ist.

10. Mikroorganismus nach Anspruch 9, der eine tierische Zelle, vorzugsweise eine Eierstockzelle des Chinesischen Hamsters (CHO-Zelle) ist.

11. Verfahren zur Herstellung eines Proteins mit der biologischen Aktivität von menschlichem Faktor VIII:C, umfassend das Züchten eines transformierten Mikroorganismus nach Anspruch 9 oder 10 unter geeigneten Bedingungen in einem Medium und das Gewinnen des Proteins aus dem Medium.

## Patentansprüche (Patentansprüche für folgende(n) Vertragsstaat(en): ES)

1. Verfahren zur Herstellung eines Plasmids zur Expression eines Proteins mit der biologischen Aktivität von menschlichem Faktor VIII:C, umfassend den Einbau eines ein Protein mit der biologischen Aktivität von menschlichem Faktor VIII:C codierenden Gens stromabwärts eines Hühner-β-Actinpromotors oder eines Derivats davon, mit Ausnahme des menschlichen β-Actinpromotors in einen Vektor.

2. Verfahren nach Anspruch 1, wobei der Hühner-β-Actinpromotor die vollständige in Figur 1 gezeigte DNA-Sequenz oder einen Teil davon mit einer Promotoraktivität enthält.

3. Verfahren nach Anspruch 1, wobei das Derivat des Hühner-β-Actinpromotors ein DNA-Fragment des Hühner-β-Actinpromotors ist, das an seinem 3'-Ende mindestens die Nucleotide bis zum C an der Position -5 enthält, berechnet von dem Initiationscodon ATG des ursprünglichen β-Actinstrukturgens.

4. Verfahren nach einem der Ansprüche 1 bis 3, wobei der Vektor außerdem mindestens einen Replikationsursprung (ori) umfaßt.

5. Verfahren nach Anspruch 4, wobei der ori von SV40 stammt.

6. Verfahren nach einem der Ansprüche 1 bis 5, wobei eine ein Signalpeptid codierende DNA-Sequenz an das das Protein mit der biologischen Aktivität von menschlichem Faktor VIII:C codierende Gen geknüpft ist.

7. Verfahren nach einem der Ansprüche 1 bis 6, wobei das Protein mit der biologischen Aktivität von menschlichem Faktor VIII:C ein Protein ist, bei dem der Arg-Rest an Position 740 am Aminoterminus der H-Kette von menschlichem Faktor VIII:C mit dem Glu-Rest an Position 1649 am Carboxyterminus der L-Kette von menschlichem Faktor VIII:C kovalent verknüpft ist.

8. Verfahren nach einem der Ansprüche 1 bis 7, wobei der Vektor außerdem ein amplifizierbares Gen umfaßt.

9. Mikroorganismus, der mit einem Expressionsplasmid, erhältlich nach einem der Ansprüche 1 bis 8, transformiert ist.

10. Mikroorganismus nach Anspruch 9, der eine tierische Zelle, vorzugsweise eine Eierstockzelle des Chinesischen Hamsters (CHO-Zelle) ist.

11. Verfahren zur Herstellung eines Proteins mit der biologischen Aktivität von menschlichem Faktor VIII:C, umfassend das Züchten eines transformierten Mikroorganismus nach Anspruch 9 oder 10 unter geeigneten Bedingungen in einem Medium und das Gewinnen des Proteins aus dem Medium.

## Revendications (Revendications pour l'(les) Etat(s) contractant(s) suivant(s): AT, BE, CH, DE, FR, GB, IT, LI, LU, NL, SE)

1. Plasmide pour l'expression d'une protéine ayant l'activité biologique du facteur VIII:C humain, qui comprend :
(a) un promoteur de la β-actine de poulet ou un dérivé de celui-ci, à l'exclusion du promoteur de la β-actine humaine, et
(b) en aval du promoteur (a), un gène codant ladite protéine ayant l'activité biologique du facteur VIII:C humain.

2. Plasmide selon la revendication 1, dans lequel ledit promoteur de la β-actine de poulet contient la séquence d'ADN complète représentée sur la figure 1 ou une partie de celle-ci ayant une activité de promoteur.

3. Plasmide selon la revendication 1, dans lequel le dérivé du promoteur de la β-actine de poulet est un fragment d'ADN du promoteur de la β-actine de poulet contenant à son extrémité 3' au moins les nucléotides jusqu'à C en position -5 calculée à partir du codon d'initiation ATG du gène de structure de la β-actine originel.

4. Plasmide selon l'une quelconque des revendications 1 à 3, qui comprend en outre au moins une origine de réplication (ori).

5. Plasmide selon la revendication 4, dans lequel la ori est dérivée de SV40.

6. Plasmide selon l'une quelconque des revendications 1 à 5, dans lequel une séquence d'ADN codant un peptide signal est liée au gène codant la protéine ayant l'activité biologique du facteur VIII:C humain.

7. Plasmide selon l'une quelconque des revendications 1 à 6, dans lequel la protéine ayant l'activité biologique du facteur VIII:C humain est une protéine dans laquelle le résidu Arg en position 740 à l'extrémité amino de la chaîne H du facteur VIII:C humain est lié de manière covalente au résidu Glu en position 1649 à l'extrémité carboxyle de la chaîne L du facteur VIII:C humain.

8. Plasmide selon l'une quelconque des revendications 1 à 7, qui comprend en outre un gène amplifiable.

9. Micro-organisme qui est transformé avec un plasmide d'expression selon l'une quelconque des revendications 1 à 8.

10. Micro-organisme selon la revendication 9, qui est une cellule animale, de préférence une cellule ovarienne de hamster chinois (CHO).

11. Procédé de production d'une protéine ayant l'activité biologique du facteur VIII:C humain, qui comprend la culture d'un micro-organisme transformé selon la revendication 9 ou 10 dans des conditions appropriées dans un milieu et la récupération de ladite protéine à partir de la culture.

## Revendications (Revendications pour l'(les) Etat(s) contractant(s) suivant(s): ES)

1. Procédé de construction d'un plasmide pour l'expression d'une protéine ayant l'activité biologique du facteur VIII:C humain, qui comprend l'incorporation dans un vecteur d'un gène codant une protéine ayant l'activité biologique d'un facteur VIII:C humain en aval d'un promoteur de la β-actine de poulet ou d'un dérivé de celui-ci, à l'exclusion du promoteur de la β-actine humaine.

2. Procédé selon la revendication 1, dans lequel ledit promoteur de la β-actine de poulet contient la séquence d'ADN complète représentée sur la figure 1 ou une partie de celle-ci ayant une activité de promoteur.

3. Procédé selon la revendication 1, dans lequel le dérivé du promoteur de la β-actine de poulet est un fragment d'ADN du promoteur de la β-actine de poulet contenant à son extrémité 3' au moins les nucléotides jusqu'à C en position -5 calculée à partir du codon d'initiation ATG du gène de structure de la β-actine originel.

4. Procédé selon l'une quelconque des revendications 1 à 3, dans lequel le vecteur comprend en outre au moins une origine de réplication (ori).

5. Procédé selon la revendication 4, dans lequel la ori est dérivée de SV40.

6. Procédé selon l'une quelconque des revendications 1 à 5, dans lequel une séquence d'ADN codant un peptide signal est liée au gène codant la protéine ayant l'activité biologique du facteur VIII:C humain.

7. Procédé selon l'une quelconque des revendications 1 à 6, dans lequel la protéine ayant l'activité biologique du facteur VIII:C humain est une protéine dans laquelle le résidu Arg en position 740 à l'extrémité amino de la chaîne H du facteur VIII:C humain est lié de manière covalente au résidu Glu en position 1649 à l'extrémité carboxyle de la chaîne L du facteur VIII:C humain.

8. Procédé selon l'une quelconque des revendications 1 à 7, dans lequel le vecteur comprend en outre un gène amplifiable.

9. Micro-organisme qui est transformé avec un plasmide d'expression qui peut être obtenu par l'un quelconque des procédés selon les revendications 1 à 8.

10. Micro-organisme selon la revendication 9, qui est une cellule animale, de préférence une cellule ovarienne de hamster chinois (CHO).

11. Procédé de production d'une protéine ayant l'activité biologique du facteur VIII:C humain, qui comprend la culture d'un micro-organisme transformé selon la revendication 9 ou 10 dans des conditions appropriées dans un milieu et la récupération de ladite protéine à partir de la culture.
